# EUROPÄISCHE PATENTSCHRIFT

(11) **EP 0 782 383 B1**
(45) Veröffentlichungstag und Bekanntmachung des Hinweises auf die Patenterteilung: **21.10.1998**
(21) Anmeldenummer: 95933354.3
(22) Anmeldetag: 09.09.1995
(51) Int. Cl.: A01G 7/06

(54) **VERWENDUNG NADELLOSER DRUCKMITTELBETÄTIGTER INJEKTIONS-GERÄTE ZUR VERABREICHUNG VON WIRKSTOFFEN AN PFLANZEN**
USE OF NEEDLELESS PNEUMATICALLY ACTUATED INJECTION APPARATUS FOR ADMINISTERING ACTIVE SUBSTANCES TO PLANTS
UTILISATION D'APPAREILS D'INJECTION PNEUMATIQUES SANS AIGUILLE POUR ADMINISTRER DES PRINCIPES ACTIFS A DES PLANTES

(30) Priorität: 17.09.1994 DE 4433190; 31.10.1994 DE 4438990
(43) Veröffentlichungstag der Anmeldung: 09.07.1997
(73) Patentinhaber: LTS LOHMANN Therapie-Systeme GmbH, 56567 Neuwied (DE)
(72) Erfinder: HOFFMANN, Hans-Rainer, D-56566 Neuwied (DE); KLOCZKO, Malgorzata, D-53545 Linz (DE); ROREGER, Michael, D-56567 Neuwied (DE)
(74) Vertreter: Flaccus, Rolf-Dieter, Dr.
(86) Internationale Anmeldenummer: EP9503556
(87) Internationale Veröffentlichungsnummer: WO9608135

(56) Entgegenhaltungen:
- EP-A- 0 119 286
- EP-A- 0 374 413
- WO-A-87/07473
- WO-A-93/03607
- DE-A- 3 115 373
- GB-A- 1 097 644
- US-A- 3 069 809
- US-A- 3 304 655
- US-A- 3 576 276
- US-A- 4 011 685
- US-A- 4 078 087
- US-A- 4 164 093
- US-A- 4 365 440
- US-A- 4 966 581

## Beschreibung

Die Erfindung betrifft die Verwendung von nadellosen druckmittelbetätigten Injektionsgeräten zur Verabreichung von Wirkstoffen an Pflanzen.

Injektion als eine Form der Verabreichung von Wirkstoffen an Pflanzen ist seit langem bekannt. Sie ist durch den Wegfall der Abdrift- und der Umweltbelastungsproblematik den üblichen Applikationsverfahren wie Spritzen und Sprühen überlegen.

Um das Injektionsverfahren bei Pflanzen, insbesondere bei Pflanzen mit verholzten Stämmen, optimal zu gestalten, wurden im Laufe der Zeit zahlreiche Injektionsgeräte entwickelt. Von den weit verbreiteten sei als erstes das sogenannte Mauget System erwähnt, das unter anderem in Patentschriften US 3 304 655 und US 4 365 440 beschrieben ist. Die in diesen Patentschriften dargestellten Geräte unterscheiden sich zwar in Details ihrer Bauweise, doch arbeiten beide nach dem gleichen Funktionsprinzip. Es handelt sich hierbei um einen nicht nachfüllbaren Injektor zur einmaligen Dosierung (Einweg-Injektor), der aus einem mit der zu injizierenden Flüssigkeit gefüllten Container und einem in den Pflanzenstamm einzusetzenden Zulaufrohr besteht. Der Container setzt sich aus zwei Schalen zusammen, die über ein Nut- und Feder-System teleskopartig ineinander geschoben werden. Beim Zusammenpressen der beiden Baukomponenten wird der Druck der über der Flüssigkeit stehenden Gasphase erhöht. Das zu injizierende Medium wird daraufhin durch das Zulaufrohr in den Stamm der Pflanze gepreßt. Der Einsatz dieser Injektionsgeräte in der gartenbaulichen Praxis hat jedoch seine Unzulänglichkeiten offenbart: zum einen ist die Einspritzung von Flüssigkeiten mittels dieses Systems stets mit der Gefahr eines Wirkstoffverlusts (Flüssigkeitsleckage aus der Bohröffnung im Stamm) verbunden, zum anderen erfordert dieses Injektionsverfahren einen relativ großen zeitlichen und anwendungstechnischen Aufwand.

Eine brauchbare Alternative stellt der in den siebziger Jahren in den USA entwickelte Hochdruck-Injektor dar. Als Beispiel einer Patentschrift, die sich mit diesem Injektionsgerät befaßt, sei hier die US 4 011 685 genannt. Der Hauptvorteil dieses Gerätes liegt in der Minimierung der Gefahr der Flüssigkeitsleckage, die durch den Einbau einer Pumpvorrichtung sowie einer sogenannten selbstverschließenden Nadel erzielt worden ist. Beim Einsatz dieses Injektionsgerätes kommt es nämlich infolge des verwendeten hohen Drucks (bis 350 bar) zum raschen Sich-Zusammenziehen von Ligninfasern der Gewebezellen in der Nadelumgebung und somit zum dichten Verschließen des zuvor entstandenen Hohlraumes, was einen möglichen Wirkstoffaustritt verhindert. Dieses Injektionssystem eignet sich für mehrmalige Anwendung zum Einspritzen von Flüssigkeiten verschiedener Viskosität. Nachteilig ist jedoch hier die Tatsache, daß sein Einsatz in der Regel eine Vorbohrung eines Aufnahmeraumes im Stamm der Pflanze erforderlich macht. Darüber hinaus ist es denkbar, daß der Grad der Verschlußdichte der Öffnung von dem Ausmaß der Lignifizierung der Zellwände und somit von der Art der Pflanze abhängig ist. Dieser Sachverhalt bedeutet, daß eine Injektion mittels der Vorrichtung letztendlich eine potentielle Gefahr einer Flüssigkeitsleckage beinhaltet.

Die Patentpublikation US 4 078 087 offenbart als weitere Injektionstechnik ein Injektionssystem, bei dem eine gleichmäßige Versorgung des Pflanzenstammes mit dem Injektionsmedium im Vordergrund steht. Maßgeblich ist hier, daß das Injizieren der Flüssigkeit nicht in eine einzelne Kanüle, sondern in ein Ableitungssystem erfolgt. Das Ableitungssystem, in das das Injektionsmedium unter Druck aus einem Behälter gepreßt wird, besteht aus einer Kupplung, einer unterschiedlichen Anzahl von sog. 'T'-Verbindern, die in vorgebohrte Stammöffnungen eingesetzt werden, und einem Abschlußhahn. Der Einsatz von 'T'-Verbindern, die Flußweichen darstellen, bei denen ein Teil der Flüssigkeit in den Stamm injiziert und der Restteil weitergeleitet wird, gewährleistet, daß das Leitungssystem der Pflanze allseitig und gleichmäßig mit Wirkstoff-Flüssigkeit versorgt wird.
Ähnlich wie im Falle der in vorliegender Schrift bis jetzt beschriebenen Injektionsgeräte ist auch dieses System mit dem Problem des unerwünschten Austritts des injizierten Mediums behaftet.

Als Beispiel eines weiteren Injektionssystems, das aus dem Stand der Technik bekannt ist, soll hier das in der Patentpublikation US 3 576 276 dargestellte genannt werden. Bei dem dort beschriebenen Injektionsgerät wird auf das altbewährte Funktionsprinzip einer Subkutan-Spritze zurückgegriffen. Neu dagegen ist die Verwendung einer Applikationshilfe in Form eines Rohrs, das bereits vor dem Ausführen der Injektion in den Baumstamm in gewünschte Tiefe eingesetzt wird. Die Verwendung dieses zusätzlichen Zuleitungsrohrs, in dem sich die Spritznadel während der Applikation befindet, ermöglicht das Auffüllen der Nadel mit dem zu injizierenden Medium vor dem Aufsetzen des Spritzzylinders mit dem Verdrängerkolben. Diese Modifizierung der üblichen Reihenfolge der Arbeitsschritte während der Applikation erfüllt den Zweck, die zu injizierende Flüssigkeit schnell und effektiv, da luftblasenfrei, einzuspritzen.

Nachteilig bei diesem Verfahren ist jedoch der relativ große Zeit- und Arbeitsaufwand, der mit der Verwendung der Zusatz-Applikationshilfe verbunden ist.

Von den weiteren nach dem Stand der Technik beschriebenen Injektionsverfahren ist noch das in CA-PS 1 089 645 beschriebene zu nennen, das unter Verwendung eines Injektionsgerätes arbeitet. Der Gegenstand dieser Erfindung ist ein Druck-Injektor zur Anwendung bei Pflanzen, der insbesondere zum Einsatz bei der Bekämpfung von pilzlichen Gefäßkrankheiten (z.B. Ulmensterben) bei Bäumen geschaffen worden ist. Diese Vorrichtung wurde vorrangig mit dem Ziel entwickelt, Injektionen wirkstoffverlustfrei zu gestalten. Die dazu erforderliche Minimierung der Flüssigkeitsleckage wurde durch die Verwendung eines Zulaufrohrs (einer Zulaufnadel) realisiert, dessen Oberfläche gezackt bzw. gezahnt ist. Durch diese unebene Oberflächenstruktur wird ein enger Kontakt zum anliegenden Pflanzengewebe hergestellt, so daß das bereits injizierte Medium überhaupt nicht oder nur in geringem Maße zurückfließen kann.

Bei dem in US 4 164 093 beschriebenen Injektionsgerät schließlich handelt es sich um einen Mini-Druckinjektor zur Anwendung bei Pflanzentrieben von geringem Durchmesser wie z.B. bei Sämlingen, Baumzweigen oder Jungpflanzen. Das Gerät umfaßt eine Spritze (nach einem Bauprinzip einer handelsüblichen subkutanen Einwegnadel-Spritze) und eine praxisübliche Vielzweck-Gartenzange, wobei die Spritze an der feststehenden Zangenbacke starr befestigt ist. Die freie Verstellbarkeit der Backenweite ermöglicht beliebiges Positionieren des Mini-Injektors an der Sproßachse und gewährleistet eine flexible Handhabung des gesamten Gerätes in Abhängigkeit von der Art der verwendeten Pflanze.

Bei keinem der nach dem Stand der Technik bekannten Injektionsgeräte zur Anwendung an Pflanzen ist es gelungen, das Problem der Leckage des injizierten Mediums vollständig und zufriedenstellend zu lösen. Darüber hinaus erfordert der Einsatz der meisten hier angeführten Injektionssysteme einen relativ hohen apparativen und zeitlichen Aufwand. Ferner eignen sich diese Geräte lediglich zum Einspritzen von flüssigen Medien.

Da keine der bisher erwähnten Druckschriften einen Hinweis auf eine Verwendung von Injektionsgeräten bei nicht verholzten Pflanzen enthält, ist davon auszugehen, daß sie für einen derartigen Einsatz ungeeignet sind.

Es ist die Aufgabe der vorliegenden Erfindung, ein Injektionsverfahren zur Verfügung zu stellen, mit dem sowohl feste als auch flüssige Medien injiziert werden, wobei im Falle der zuletzt erwähnten der Nachteil der Leckage vermieden wird, und das darüber hinaus bei Gehölzen sowie bei Pflanzen mit nicht verholzten Sproßachsen angewendet werden kann.

Überraschenderweise wurde gefunden, daß die Verwendung nadelloser druckmittelbetätigter Injektionsgeräte gemäß den kennzeichnenden Merkmalen des Hauptanspruchs 1 und des Unteranspruchs 2 dieser Anforderung in vollem Umfang gerecht wird.

Im folgenden wird die Erfindung detailliert beschrieben.

Nadellose druckmittelbetätigte Injektionsgeräte sind aus der Humanheilkunde und Tiermedizin bekannt. Seit Jahren werden sie zu diagnostischen und therapeutischen Zwecken überall dort, wo schmerzfreies Injizieren oder Punktieren erforderlich ist, verwendet. Sie sind im Handel unter verschiedenen Namen erhältlich (hierzu sollen beispielsweise die in der ehemaligen Sowjetunion hergestellte Impfspritze (Bienchen", das in den USA entwickelte "Jet" und das ungarische Impfgerät "Viper" erwähnt werden) sowie in zahlreichen Patent- und Offenlegungsschriften (z.B. DE 34 67 301, EP 0 119 286, US 4 966 581, DE 31 15 373) ausführlich beschrieben. Bei Pflanzen haben sie jedoch bis jetzt keine Verwendung gefunden.

Es wurde überraschenderweise gefunden, daß nadellose Injektionsgeräte vom grundsätzlichen Bautyp, so wie sie in den genannten Patentschriften beschrieben sind, sich hervorragend zum Injizieren von verschiedenen Substanzen in Pflanzen eignen.

Bei den Injektionsgeräten der genannten Art handelt es sich um Vorrichtungen, bei denen das zu injizierende Medium unter Aufwand von derartiger Energie die Ausspritzkammer der Vorrichtung durch eine Düse verläßt, daß eine nadelllose Injektion möglich ist. Durch den dadurch erzeugten kurzen Druckstoß wird auf dem zuinjizierenden Objekt (Pflanzenorgan) die für das Injektionsmittel erforderliche Einschußöffnung mit gewünschter Tiefe erzeugt. Die Injektionstiefe kann dabei durch den Winkel und Abstand des Gerätes von dem zu injizierenden Objekt variiert werden: z.B. je näher die Düse der Objektoberfläche ist, desto tiefer der Injektionskegel.

Der für das Ausschießen des zu injizierenden Mediums erforderliche Druck kann je nach Bauweise des Injektionsgerätes unterschiedlich erzeugt werden. Hierzu können beispielsweise als Druckmittel aus einer Kohlensäurepatrone ausströmendes Gas (DE 34 67 301) oder eine Druckpumpe (DE 31 15 373) verwendet werden.

Der besondere Vorteil dieser Erfindung liegt darin, daß bei der Verwendung von dieser Art kanülen- und nadelfreien Injektoren die Gefahr eines durch die Leckage bedingten Wirkstoffverlustes vollständig eliminiert ist. Dies gilt insbesondere im Falle der Injektionen bei nicht verholzten Pflanzen, bei denen es aufgrund des osmotischen Drucks der Nachbarzellen sehr schnell zum Verschließen der Einschußöffnung kommt.

Die durch die Erfindung erreichten weiteren Vorteile sind darüber hinaus darin zu sehen, daß das zu injizierende Medium in fließfähiger, halbfester oder fester Form vorliegen kann. Bei solcher Art der Düseninjektionen ist es nämlich möglich, auch hochviskose Flüssigkeiten unter Einsatz eines entsprechend hohen Drucks in die Objekte einzuspritzen. Der Grund dafür liegt darin, daß Kanülen und Nadeln hier nicht verwendet werden. Dadurch wird das üblicherweise bei zähflüssigen Medien auftretende Problem des Nadelverschlusses, z.B. durch Verstopfung, ausgeschlossen.

Das nadellose Injektionsverfahren bietet auch die Möglichkeit, Medien in kristalliner Form zu injizieren. Voraussetzung dafür ist, daß einzelne Teilchen des Injektionsmittels ausreichend klein (mikrofein) dimensioniert sind. Als bevorzugte Partikelgröße ist der Bereich <1 µm zu nennen.

Die Wirkstoffe können dabei allein oder in Mischung miteinander vorliegen. Sie können im Injektionsmedium gelöst oder dispergiert sein.

Unter den Wirkstoffen, die unter Verwendung nadelloser Injektionsgeräte an Pflanzen verabreicht werden können, sind in erster Linie systemisch wirkende Pflanzenschutzmittel (Insektizide, Akarizide, Fungizide, Bakterizide) sowie Pflanzenstärkungsmittel und Wachstumsregulatoren zu nennen.

Systemische Insektizide sind beispielsweise Buthocarboxim, Dimethoat, Fenoxycarb, Methamyl, Oxamyl, Oxydemtetonmethyl, Pirimicarb oder Propoxur.

Systemische Akarizide sind beispielsweise Clofentizin, Fenbutation-oxyd und Hexythiazox.

Systemische Fungizide sind beispielsweise Benomyl, Bromuconazol, Bitertanol, Etaconazol, Flusilazol, Furalaxyl, Fosetyl-Al, Imazalil, Metalaxyl, Penconazol, Propiconazol, Thiabendazol, Triadimefon, Triadimenol oder Triforine.

Unter den systemischen Bakteriziden ist beispielsweise Flumequine zu nennen.

Als Pflanzenstärkungsmittel sind beispielsweise Pflanzenauszüge aus Brennessel, Rainfarn, Schachtelhalm oder Staudenknöterich zu nennen. Diese bioaktiven Substanzen können je nach dem Injektionsort lokale oder systemische Wirkung in der Pflanze entfalten.

Systemische Wachstumsregulatoren sind beispielsweise Etephon und β-Indolylessigsäure (IES).

Als eine bevorzugte Anwendung der nadellosen Injektoren soll in diesem Zusammenhang die Verabreichung von wachstumshemmenden Substanzen, eine Alternative zu manuellen Erziehungs- und Schnittmaßnahmen im öffentlichen Grün, genannt werden. Auf diesem Gebiet haben Injektionssysteme in den letzten Jahren (insbesondere in den USA) an ihrer Bedeutung erheblich zugenommen.

Weitere Substanzen, die unter Verwendung nadelloser Injektionsgeräte verarbeitet werden können, sind Holzschutz- und Holzimprägniermittel.

Seit Jahrhunderten wird Nutzholz zum Schutz gegen Alterungs- und Witterungseinflüsse mit Öl- und Wachsgemischen oder sonstigen Chemikalien (z.B. gegen holzfressende Insekten) getränkt oder mit filmbildenden Überzügen versehen.

Diese Behandlungen haben jedoch den Nachteil, daß sie von Zeit zu Zeit erneuert werden müssen.

Verwendung nadelloser Hochdruckinjektoren bietet eine günstige Alternative zu dem konventionellen Imprägnierverfahren.
Bedingt durch die Tatsache, daß mittels dieser Vorrichtung relativ hochviskose Medien injiziert werden können, ist es möglich, unter Einsatz der genannten Geräte auch polymerhaltige Flüssigkeiten in ein zu behandelndes Stück Holz einzuspritzen.
Dies ist insofern von besonderer Bedeutung, als das Injektionsmedium eine Formulierung mit gesteuerter Wirkstofffreisetzung sein kann. Da es möglich ist, mittels dieser Art der Wirkstoffzubereitungen das Freisetzungsprofil (Freisetzungsdauer und -geschwindigkeit) zu bestimmen, bietet somit das nadellose Injektionsverfahren den Vorteil eines langfristigen Schutzes der Objekte.

Folglich eignen sich nadellose Injektionsverfahren bevorzugt bei der Behandlung von Holzgegenständen, bei denen ein langfristiger und anhaltender Schutz gefordert wird, z.B. bei Holzelementen von Pflanzenunterstützungseinrichtungen (Erziehungsgerüste, Baumpfähle) im Erwerbsgartenbau.

Ein weiterer wichtiger Vorteil, der bei der Verwendung nadelloser Injektionsgeräte erzielt wird, ist eine Arbeitszeitersparnis. Diese Vorrichtungen erlauben ein schnelles Arbeiten, sind robust und relativ langlebig. Da sie darüber hinaus zur Sterilisation im Autoklaven geeignet sind, können sie auch in Fällen der Wirkstoffverabreichung verwendet werden, wo Virusfreiheit bzw. Virusinfektionsgefahr von Bedeutung sind, z.B. bei der Züchtung von virusfreien Sorten oder bei der Meristemvermehrung.

Die Erfindung wird anhand der folgenden Beispiele näher erläutert:

### Beispiel 1

2,5 Gewichtsanteile pulverförmiger Polyacrylsäure (Carbopol ETD 2050) wurden in 97 Gewichtsanteilen Wasser dispergiert. Der so hergestellten Suspension wurden 0,5 Gewichtsanteile des Wirkstoffs Al-Fosetyl zugesetzt. Der zugegebene Wirkstoff wurde unter ständigem Rühren in der Masse homogen verteilt. Anschließend wurde die wirkstoffhaltige wäßrige Acrylsäure-Dispersion mit einer relativen Viskosität von 0,86 Pa·s (bei 25 °C nach Brookfield LVF/Meßkörper) in ein druckmittelbetätigtes nadelloses Injektionsgerät (Typ Dermo-Jet) abgefüllt und an der Basis eines einjährigen Himbeertriebs (Rubus idaeus) mit einem Druck von 8,1 bar in Pflanzengewebe eingespritzt.

### Beispiel 2

0,9 g Salicylsäure (Pflanzenresistenzinduktor) wurden unter Erwärmung auf 30 °C in 500 ml dest. Wasser gelöst. 100 ml der so erhaltenen Lösung wurden in eine druckmittelbetätigtes nadelloses Injektionsgerät (Typ Dermo-Jet) abgefüllt und an der Basis der Sproßachse einer Tabak-Pflanze (Nicotiana tabakum) in Pflanzengewebe eingespritzt.

## Patentansprüche

1. Verwendung nadelloser druckmittelbetätigbarer Injektionsgeräte vom grundsätzlichen Bautyp, wie sie beim Stand der Technik aus der Humanheilkunde und Tiermedizin zu diagnostischen oder therapeutischen Zwecken bekannt und im Gebrauch sind, zur Injektion einer in fester, halbfester oder flüssiger Form vorliegenden Formulierung in Pflanzen zur steuerbaren Freisetzung eines Mediums aus der Gruppe systemisch wirkender Pflanzenschutzmittel, Pflanzenstärkungsmittel, Wachsumtsregulatoren, Düngemittel sowie Holzbehandlungsmittel.

2. Verwendung nach Anspruch 1 zur Injektion eines Mediums in Form von Pellets mit einem Durchmesser von 0,1 bis 5,0 µm, vorzugsweise von 0,2 bis 1,0 µm.

## Claims

1. The use of needle-free pressure-actuated injection devices of the basic construction type as known and used according to the prior art in human medicine and veterinary medicine for diagnostic or therapeutic purposes, for injection in plants of a formulation present in solid, semi-solid or liquid form, for the controlled release of a medium of the group of systemically active plant protection agents, plant restoratives, growth regulators, fertilizers and preparations for wood treatment.

2. The use according to claim 1 for the injection of a medium in the form of pellets having a diameter of 0.1 to 5.0 µm, preferably 0.2 to 1.0 µm.

## Revendications

1. Utilisation d'appareils d'injection pneumatiques sans aiguille du type de construction fondamental tels qu'ils sont connus et utilisés suivant l'état de la technique dans le domaine de la médecine humaine et vétérinaire à des fins diagnostiques ou thérapeutiques, pour l'injection d'une formulation se trouvant sous forme solide, semi-solide ou liquide à des plantes en vue de la libération contrôlable d'un milieu choisi dans le groupe des produits phytosanitaires, des reconstituants pour plantes, des régulateurs de la croissance, des engrais, de même que des produits pour le traitement du bois, à action systémique.

2. Utilisation selon la revendication 1 pour l'injection d'un milieu sous forme de pellets avec un diamètre de 0,1 à 5,0 µm, de préférence de 0,2 à 1,0 µm.
